# EUROPEAN PATENT SPECIFICATION

(11) **EP 1 534 721 B1**
(45) Date of publication and mention of the grant of the patent: **26.07.2006**
(21) Application number: 03764062.0
(22) Date of filing: 07.07.2003
(51) Int. Cl.: C07F 9/6518, A61K 31/675, F26B 5/06

(54) **PROCESS FOR CONTROLLING THE HYDRATE MIX OF THE DISODIUM SALT OF FOSFLUCONAZOLE**
VERFAHREN ZUR EINSTELLUNG DER HYDRATMISCHUNG DES FOSFLUCONAZOLE-DINATRIUMSALZES
PROCEDE DE REGULATION DU MELANGE D'HYDRATES DU SEL DISODIQUE DU FOSFLUCONAZOLE

(30) Priority: 16.07.2002 GB 0216515
(43) Date of publication of application: 01.06.2005
(73) Proprietor: Pfizer Limited, Sandwich, Kent CT13 9NJ (GB); PFIZER INC., New York, N.Y. 10017 (US)
(72) Inventor: AUFFRET, Anthony D., Pfizer Global Res. & Dev., Sandwich, Kent CT13 9NJ (GB); FITZGERALD, Michael P., Pfizer Global Res. & Dev., Sandwich, Kent CT13 9NJ (GB)
(74) Representative: Lane, Graham Mark Hamilton
(86) International application number: PCT/IB2003/003119
(87) International publication number: WO 2004/007507

(56) References cited:
- EP-A- 0 327 081
- US-A- 3 178 829
- US-A- 3 487 554
- G. ZOGRAFI ET AL.: "Stimuli to the revision process" PHARM. FORUM, 1991, pages 1459-1474, XP009020377

## Description

This invention relates to a process for controlling the hydrate mix of the disodium salt of fosfluconazole, or a composition comprising said compound, the compound being capable of forming a plurality of hydration forms of differing stability and also of dissolution to give a solution that, when frozen below the eutectic point, is a eutectic mixture.

The disodium salt of fosfluconazole (hereinafter DSFF) is disclosed in WO97/28169 and has the following structure:

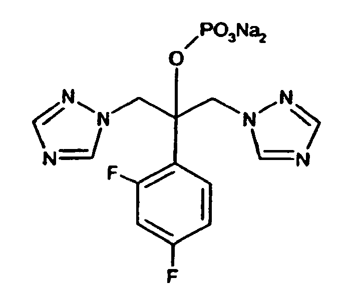

A number of hydration states of DSFF have now been found to exist and it is hypothesised that these are the dodecahydrate (33.4% w/w water), hexahydrate (20.1 % w/w water), trihydrate (11.2% w/w water) and monohydrate (4.0% w/w water) forms. The anhydrous form of DSFF is believed to be amorphous. While the tri- and hexahydrate forms of DSFF are both chemically and thermally stable, it has been found that certain hydrate forms, such as the dodecahydrate, exhibit physical and/or chemical instability. While not wishing to be bound to any particular theory, it is believed that the eutectic form of DSFF is a dodecahydrate and it has been found that this dodecahydrate form is thermally unstable. Furthermore, it has been discovered that forms of DSFF which have a water content of from 4.0% w/w to 11.2% w/w are also chemically unstable. It is hypothesised that such a hydrate mix is a combination of tri- and monohydrate forms. Additionally, it has been found that samples of DSFF having a water content above the hexahydrate stoichiometry (20.1% w/w) collapse in a manner consistent with equilibration to the hexahydrate and water. Indeed, any composition comprising an unstable hydrate form, despite possibly containing stable forms, will decompose.

A stable hydration form of DSFF may be obtained by crystallisation from either acetonitrile/water or isopropanol/water mixtures. However, in order for the resulting product to be useful as a drug it must be sterile, but sterile recrystallisation is neither trivial nor economical.

Lyophilisation is a well known technique for the stabilisation of labile products which would otherwise be susceptible to biological or chemical degradation. The process has established itself as the standard method for the stabilization of many drug substances in the solid state to produce products of superior quality and stability.

It is well known in the art that for lyophilisation to take place a solution must be cooled to a temperature below which further cooling does not affect the phase composition, the so called eutectic temperature. Classical textbook models suggest that thermodynamic equilibrium eutectic freezing is universal. However, it is recognised in the art that it only occurs in the minority of frozen solutions and, actually, in the majority of cases a glass is formed. F. Franks, Cryo-Letters, 11., 93-110, 1990 teaches that the events taking place in a solution during the freezing process can seldom be predicted from equilibrium phase diagrams. In practice the solutes either incompletely precipitate or do not precipitate at all.

DSFF, however, is an example of a compound which is unusual, in that upon freezing it does conform to the classical models and precipitates from solution.

A typical freeze-drying process involves reducing the temperature of a compound, or a composition thereof, to below its "collapse" temperature. For a compound or composition thereof which undergoes crystallisation upon cooling, i.e. which behaves according to classical models, the collapse temperature will be the eutectic temperature. For a compound or a composition thereof which does not crystallise, or does so only partially, the collapse temperature will be the glass transition temperature of the freeze concentrate. Once the appropriate temperature conditions have been applied, the pressure in the apparatus is reduced from atmospheric pressure to from 83.3 Pa to 5.3 Pa, in order to sublime the ice from the frozen product. This is the primary drying phase. Ideally the temperature of the shelves in the apparatus should be kept below the collapse temperature of the compound or composition until all of the ice has been removed. However, it is common practice to increase the temperature in an attempt to speed up the process. In this situation, the shelf temperature must be kept to a level such that the heat coming into the system is balanced by the heat and mass transfer leaving the system, in order that the product never warms above its temperature of collapse. For the majority of compounds or compositions thereof, i.e. those which do not behave according to classical models, if the primary drying phase is extended beyond the time at which sublimation of the ice has been completed there is little effect. Once the primary drying phase has been completed, an amorphous compound will still typically contain a substantial amount of water (e.g. about 30% to about 50% by weight). Therefore, a secondary drying phase is undertaken to effect complete dehydration of the compound. The secondary drying process involves increasing the temperature of the shelves in the apparatus from below the collapse temperature to from 25°C to 30°C.

However, it has been discovered that some compounds, and compositions thereof, more particularly those exhibiting a plurality of hydration forms of differing stability, may be deleteriously affected by being subjected to such a secondary drying phase. For example, when DSFF is subjected to a typical freeze-drying process a product is produced which has a water content of 1.2% w/w, i.e. below its monohydrate stoichiometry (4.0% w/w water). This form of DSFF is chemically unstable and degrades. Furthermore, it has been discovered that forms of DSFF which have a water content of from 4.0% w/w to 11.2% w/w are also chemically unstable. It is hypothesised that such a hydrate mix is a combination of tri- and monohydrate forms. Additionally, it has been found that samples of DSFF having a water content above the hexahydrate stoichiometry (20.1% w/w) collapse in a manner consistent with equilibration to the hexahydrate and water.

The hydrate mix of a compound could, in principle, be controlled by a process which controls the Relative Humidity (RH) to which the compound is exposed. Such a process would comprise subjecting the compound or composition to a standard drying cycle in a freeze-drying apparatus followed by rehydration by providing a gas having an appropriately selected RH at a controlled temperature. However, in practice there would be a multitude of significant problems associated with such a process. Firstly, as discussed above, it is vital that compounds and compositions are prepared in sterile form when they are to be used for pharmaceutical purposes. This results in the significant practical problem of ensuring that the moist rehydration gas does not lead to corrosion or microbial contamination of the associated delivery system, which would severely impact on the economic viability of such a process. Furthermore, the rate of rehydration of the intermediate might not be sufficiently high to afford an economically viable process. In addition, it is possible that the compound or composition would be dried to the point that an unstable intermediate might be formed, which would mean that the end product of the process would be contaminated and therefore unacceptable for pharmaceutical use.

The apparent instability of compounds having a mixture of unstable and stable hydration states (e.g. the compound DSFF as a mixture of mono- and tri-hydrates) limits their potential utility as drugs owing to poor shelf life and there is a need in the art, therefore, for a process of producing a stable hydrate mix of such compounds. In particular there is a need in the art for a process for producing a mixture of the tri- and hexahydrate forms of DSFF.

Surprisingly, we have now found that a compound having a mixture of unstable and stable hydration states can be treated by the process of the current invention to provide a corresponding hydrate mix which is both chemically and thermally stable and can be prepared in a reproducibly facile and economically viable manner.

The present invention provides a process for the preparation of a stable hydrate mix of the disodium salt of fosfluconazole, or a composition comprising said compound, the compound being capable of forming a plurality of hydration forms of differing stability and of dissolution to give a solution that, when frozen below the eutectic point, is a eutectic mixture, comprising:
a) providing a quantity of an aqueous mixture containing the compound or composition thereof in a suitable vessel in a freeze-drying apparatus;
b) reducing the temperature in the apparatus to bring about freezing and eutectic solidification;
c) reducing the pressure in the apparatus to below the saturation vapour pressure (SVP) of water over ice at the temperature of the ice;
d) maintaining the apparatus at a pressure below the SVP and, optionally, increasing the temperature in the apparatus to facilitate sublimation, until all of the ice has been sublimed;
e) maintaining the apparatus at the pressure and temperature conditions according to step d) until the desired water content has been obtained; and
f) either
   increasing the pressure in the apparatus to from 60% to 100% of atmospheric pressure (60.8 kPa to 101.3 kPa) and subsequently adjusting the temperature in the apparatus to from 5°C to 30°C;
      or
   adjusting the temperature in the apparatus to from 5°C to 30°C and subsequently increasing the pressure in the apparatus to from 60% to 100% of atmospheric pressure (60.8 kPa to 101.3 kPa).

Water in a solution will freeze at temperatures of from -2°C to -15°C. Eutectic solidification is key to the invention; that is, all of the bulk, or freezable, water in the samples of the disodium salt of fosfluconazole contained within the freeze-drier apparatus must be frozen during step b). Unfortunately it is not possible to measure the exact point at which freezing is completed within the samples, nor is it possible to measure the exact point when the product crystallises. Preferably, therefore, the temperature selected in step b) is from -10°C to 50°C, more preferably from -20°C to -40°C and most preferably the temperature is -30°C, to ensure that freezing and crystallisation are completed.

The pressure selected in step c) should be below the SVP of water over ice at the temperature of the ice, i.e. at the disodium salt of fosfluconazole or composition temperature. If a pressure above the SVP is used then there is a significant reduction in the driving force present to encourage the ice to sublime because the gas phase is saturated. As the pressure is lowered the driving force is increased, but if the pressure is too low the drying rate slows. This is because the heat transfer in the drier is mainly through the gas phase, even at reduced pressures. Therefore, at very low pressures the rate of heat input is decreased resulting in the reduction in the drying rate. Preferably, therefore, the pressure selected in step c) is from 1 to 250Pa, more preferably from 2 to 125Pa and most preferably 4 to 75Pa (e.g. 8Pa)

Subliming water molecules remove energy, which results in a decrease in temperature of the ice within the disodium salt of fosfluconazole or composition during the primary drying phase of the process. If the rate of sublimation of the ice from the sample is to be increased, the temperature must also be increased, as optionally indicated in step d) of the process. Whether the temperature of step d) represents an increase or not will, necessarily, depend on the temperature selected during step b), but typically the temperature selected in step d) is from -50°C to 50°C, such as from -25°C to 0°C, preferably -15°C. A higher temperature than 50°C must not be selected because there is a danger that the ice will not sublime but will pass into the liquid phase.

The apparatus is maintained at the conditions selected in step d) in order to remove the ice from the disodium salt of fosfluconazole. However, as mentioned above, it has been discovered that certain compounds will continue to dehydrate, if maintained under reduced pressure conditions, once all of the ice has sublimed and this can have a deleterious affect on the quality of the resulting product. It is known in the art that a standard pressure rise test (PRT) will indicate the point of completion of the primary drying cycle (i.e. the sublimation stage). However, for compounds that continue to dehydrate once the sublimation has been completed, the rate at which water leaves the crystal structure may be sufficiently high that the PRT is passed significantly later than would be expected from sublimation of ice alone. Preferably the drying process is monitored at intervals of one hour. Once the PRT has been passed the compounds may continue to dehydrate and an additional drying period may be required to give a product of the desired water content. This is the dehydration phase (e), which immediately follows the sublimation phase.

The duration of dehydration phase (e) depends upon the disodium salt of fosfluconazole or composition, the vial diameter and fill volume and the type of drier used. The dehydration phase removes sufficient water from the disodium salt of fosfluconazole to encourage formation of stable hydration forms and prevent the formation of unstable hydration forms. Should the dehydration phase be allowed to continue for longer than necessary, unstable hydration forms of the disodium salt of fosfluconazole may be obtained, leading to instability. Typically the duration of the dehydration phase will be from 0 to 100 hours, such as 0 to 50 hours, preferably 6 to 30 hours, for example 8, 12 or 28 hours.

With respect to step f), an increase in the pressure in the freeze-drying apparatus slows the dehydration phase sufficiently that the temperature may be increased to facilitate stoppering of the vials, thus allowing a stable hydrate mix to be achieved, although the order of these steps is not critical. Stoppering is conveniently carried out at 5°C and 88% atmospheric pressure (i.e. 89.2kPa).

Preferably the final water content of the stable hydrate mix of DSFF is from 11 % to 20% w/w, more preferably, from 14% to 17% w/w, most preferably from 15% to 16% w/w, such as 15 %.

By way of illustration only, examples of the process conditions used to prepare a stable hydrate mix of DSFF will now be described. All examples were run in a Virtis Pilot Drier having an eight square foot shelf area and a condenser temperature of -56°C. 7.5 mTorr is equivalent to 1 Pa.

### Example 1

Vials (1320) having an internal diameter of 21.35 mm and a neck diameter of 20 mm were filled with 1.6 ml of a solution comprising 100.88 mg/ml fosfluconazole in NaOH/citric acid, adjusted to pH 9.05, thereby providing 179.8 mg of DSFF. The vials were placed in the drier and frozen at a shelf temperature of -30 °C for 3 hours. The shelf temperature was then increased to -15 °C and the pressure reduced to 8 Pa to facilitate sublimation. The PRT was passed at approximately 26 h after the start of ice sublimation and the samples dried for a further 12 hours before terminating the cycle by stoppering at 5 °C and 89.2 kPa pressure. The product was a stable hydrate mix with a mean water content of 11.7 % w/w.

| | **Temperature/°C** | **Pressure/mTorr** | **Time/hrs** |
|---|---|---|---|
| **Loading** | 5 | | |
| **Freezing** | 5 | | 0.50 |
| | -30 | | 1.47 |
| | -30 | | 4.47 |
| **Evacuation** | -30 | 60 | 4.97 |
| **Primary Drying** | -30 | 60 | 5.13 |
| | -15 | 60 | 5.55 |
| | -15 | 60 | 7.55 |
| | -15 | 60 | 17.55 |
| | -15 | 60 | 22.55 |
| | -15 | 60 | 25.55 |
| | -15 | 60 | 26.55 |
| | -15 | 60 | 27.55 |
| | -15 | 60 | 28.55 |
| | -15 | 60 | 29.55 |
| | -15 | 60 | 30.55 |
| **Dehydration Phase** | -15 | 60 | 42.55 |

### Example 2

Vials (1173) having an internal diameter of 22.6 mm and a neck diameter of 20 mm were filled with 2.8 ml of a solution comprising 100.88 mg/ml fosfluconazole in NaOH/citric acid, adjusted to pH 9.05, thereby providing 314.6 mg of DSFF. The vials were placed in the drier and frozen at a shelf temperature of -30 °C for 3 hours. The shelf temperature was then increased to -15 °C and the pressure reduced to 8 Pa to facilitate sublimation. The PRT was passed at approximately 47 hours after the start of ice sublimation and the samples dried for a further 12 hours before terminating the cycle by stoppering at 5 °C and 89.2 kPa pressure. The product was a stable hydrate mix with a mean water content of 14.6 % w/w.

| | **Temperature/°C** | **Pressure/mTorr** | **Time/hrs** |
|---|---|---|---|
| **Loading** | 5 | | |
| **Freezing** | 5 | | 0.50 |
| | -30 | | 1.47 |
| | -30 | | 4.47 |
| **Evacuation** | -30 | 60 | 4.97 |
| **Primary Drying** | -30 | 60 | 5.13 |
| | -15 | 60 | 5.55 |
| | -15 | 60 | 7.55 |
| **PRT Intervals** | -15 | 60 | 17.55 |
| | -15 | 60 | 27.55 |
| | -15 | 60 | 37.55 |
| | -15 | 60 | 47.55 |
| | -15 | 60 | 49.55 |
| | -15 | 60 | 50.55 |
| | -15 | 60 | 51.55 |
| **Dehydration Phase** | -15 | 60 | 63.55 |

### Example 3

Vials (1173) having an internal diameter of 22.6 mm and a neck diameter of 20 mm were filled with 5.4 ml of a solution comprising 100.88 mg/ml fosfluconazole in NaOH/citric acid, adjusted to pH 9.05, thereby providing 606.8 mg of DSFF. The shelf temperature was then increased to -15 °C and the pressure reduced to 8 Pa to facilitate sublimation. The PRT was passed at approximately 96 h after the start of ice sublimation and the samples dried for a further 12 hours before terminating the cycle by stoppering at 5 °C and 89.2 kPa pressure. The product was a stable hydrate mix with a mean water content of 16.9 % w/w.

| | **Temperature/°C** | **Pressure/mTorr** | **Time/hrs** |
|---|---|---|---|
| **Loading** | 5 | | |
| **Freezing** | 5 | | 0.5 |
| | -30 | | 1.47 |
| | -30 | | 4.47 |
| **Evacuation** | -30 | 60 | 4.97 |
| **Primary Drying** | -30 | 60 | 5.13 |
| | -15 | 60 | 5.55 |
| | -15 | 60 | 7.55 |
| | -15 | 60 | 17.55 |
| | -15 | 60 | 27.55 |
| | -15 | 60 | 37.55 |
| | -15 | 60 | 47.55 |
| | -15 | 60 | 57.55 |
| | -15 | 60 | 67.55 |
| | -15 | 60 | 77.55 |
| | -15 | 60 | 87.55 |
| | -15 | 60 | 88.55 |
| | -15 | 60 | 89.55 |
| | -15 | 60 | 90.55 |
| | -15 | 60 | 91.55 |
| | -15 | 60 | 92.55 |
| | -15 | 60 | 93.55 |
| | -15 | 60 | 94.55 |
| | -15 | 60 | 95.55 |
| | -15 | 60 | 96.55 |
| | -15 | 60 | 97.55 |
| | -15 | 60 | 98.55 |
| | -15 | 60 | 99.55 |
| | -15 | 60 | 100.55 |
| **Dehydration Phase** | -15 | 60 | 112.55 |

In the Examples that follow, Powder X-ray diffraction patterns were determined using a Bruker-AXS Ltd D8 Advance powder X-ray diffractometer fitted with a theta-theta goniometer, Gobel mirror and a position-sensitive detector. The samples were prepared for analysis by placing the powder on to silicon wafer specimen mounts. Each specimen was irradiated with copper K-alpha₁ X-rays (wavelength = 1.5406 A) with the X-ray tube operated at 40kV/40mA. The analyses were performed with the goniometer running in continuous mode set for a 0.3 second count per 0.014° step over a two theta range of 4° to 35° 20.

### Example 4

Vials (582), 10 ml Type 1 Clear glass with a 20 mm neck diameter were filled with 2.9 ml of a solution comprising 100.88 mg/ml fosfluconazole in NaOH/citric acid adjusted to pH 9.0, thereby providing 325.8 mg of DSFF. The vials were placed in the drier and frozen at a shelf temperature of -30 °C for 3.5 hours. The shelf temperature was then increase to -15 °C and the pressure reduced to 6 Pa to facilitate sublimation. After 58.3 hours of sublimation the cycle was terminated by stoppering at 5 °C and 96 kPa pressure.

| | **Temperature/°C** | **Pressure/mTorr** | **Time/hrs** |
|---|---|---|---|
| **Loading** | 5 | | |
| **Freezing** | -30 | | 3.00 |
| | -30 | | 3.50 |
| **Evacuation** | -30 | 45 | 4.25 |
| **Primary Drying/Dehydration** | -30 | 45 | 4.50 |
| | -30 | 45 | 5.00 |
| | -15 | 45 | 50.00 |
| | -15 | 45 | 63.30 |

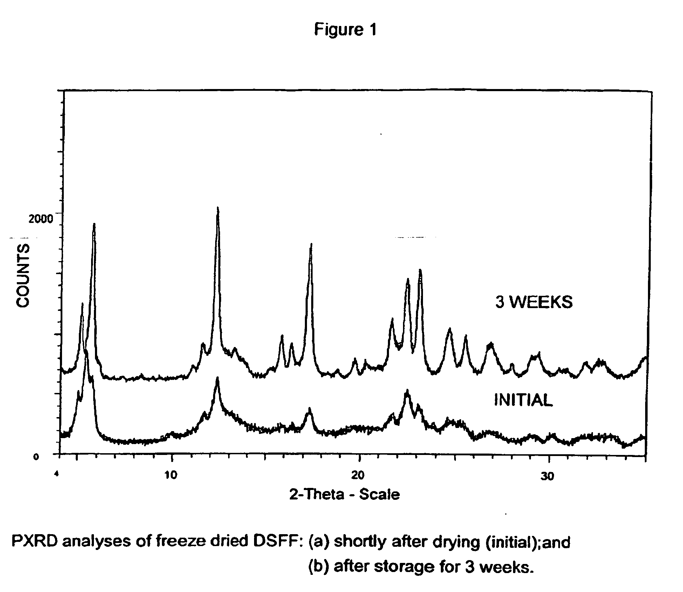

The product from the above process is found to have a water content consistent with a stoichiometry between the hexa- and trihydrate forms. Powder X-ray Diffraction (PXRD) analysis of the product suggests that immediately after processing it has little long-range order, but that it does, however, have a high degree of crystallinity, see Figure 1. The crystallinity of the sample is seen to increase after a storage period of several weeks. The PXRD pattern of the sample can be interpreted as a mixture of tri- and hexahydrate forms of DSFF. This mixture is both chemically and thermally stable. The PXRD data corresponding to Figure 1 follow:

**PXRD "INITIAL"**

| 2-Theta ° | Intensity % | 2-Theta ° | Intensity % | 2-Theta ° | Intensity % |
|---|---|---|---|---|---|
| 5.011 | 64.3 | 15.826 | 32 | 26.768 | 29.1 |
| 5.412 | 100 | 17.336 | 45.8 | 29.115 | 24.1 |
| 5.7 | 75.3 | 21.63 | 41.5 | 30.194 | 24.1 |
| 9.959 | 25.7 | 22.456 | 64.1 | 31.874 | 24.1 |
| 11.005 | 32 | 23.089 | 50.2 | 33.175 | 24.1 |
| 11.545 | 42.3 | 24.802 | 37.4 | 34.824 | 24.9 |
| 12.33 | 75.2 | | | | |

**PXRD "3 WEEKS"**

| 2-Theta ° | Intensity % | 2-Theta ° | Intensity % | 2-Theta ° | Intensity % |
|---|---|---|---|---|---|
| 5.062 | 47.9 | 17.186 | 77.5 | 26.737 | 25.1 |
| 5.631 | 91.8 | 18.776 | 11.3 | 27.91 | 13.9 |
| 6.026 | 17.3 | 19.664 | 16.5 | 28.988 | 18.6 |
| 10.91 | 13.4 | 20.235 | 14.7 | 29.305 | 20.3 |
| 11.449 | 25.5 | 21.545 | 38.5 | 30.733 | 10.8 |
| 12.164 | 100 | 22.317 | 59.3 | 31.811 | 15.2 |
| 13.162 | 24.2 | 23 | 66 | 32.604 | 15.2 |
| 15.711 | 29 | 24.56 | 32.8 | 34.792 | 19.5 |
| 16.27 | 25.5 | 25.415 | 28.5 | | |

Not wishing to be bound by any particular theory, it is thought that a significant change in structure, as might on first inspection be implied by the PXRD analysis, does not actually occur; the molecules within the sample are thought to be in a crystalline array. Rather, removal of water molecules during the drying process results in disordered distribution of those water molecules remaining in the sample and, it is believed, it is this disordered distribution of water molecules which disrupts the repetitive long range repeating order (Figure 1 - INITIAL). Over time the system equilibrates such that the water molecules redistribute and their resulting distribution no longer disrupts the repetitive long range repeating order (Figure 1 - 3 WEEKS).

### Example 5 - DSFF Trihydrate

### (a) DSFF hexahydrate

Fosfluconazole (40g) was slurried in water (120 ml) and sodium hydroxide (7-8 ml of a 47% solution, circa 4.9-5.6g NaOH) added stepwise, under nitrogen until a solution formed. The temperature was maintained between ambient and 30°C. The solution was filtered. Sodium hydroxide (42 ml of a 47% solution, circa 29.6g NaOH) was added to the filtrate until precipitation occurred. The mixture was granulated for 4 hours, filtered under vacuum and dried *in vacuo* at 50°C to afford the title compound (23.79g). PXRD:

| 2-Theta ° | Intensity % | 2-Theta ° | Intensity % | 2-Theta ° | Intensity % |
|---|---|---|---|---|---|
| 4.938 | 8.3 | 19.756 | 5.4 | 28.6 | 17.6 |
| 9.969 | 7 | 20.14 | 30.8 | 28.722 | 19.8 |
| 11.038 | 10.1 | 20.709 | 11.6 | 29.092 | 29.5 |
| 12.114 | 12.9 | 21.625 | 44.4 | 29.986 | 35.7 |
| 12.383 | 9 | 21.924 | 30.9 | 30.45 | 15.7 |
| 13.0 | 7.2 | 22.269 | 84.6 | 30.636 | 18.3 |
| 13.226 | 17.1 | 22.465 | 100 | 31.503 | 29.5 |
| 14.608 | 3.6 | 23.113 | 20.6 | 31.91 | 32.7 |
| 15.035 | 23 | 23.604 | 48.4 | 31.997 | 30 |
| 15.579 | 28.3 | 24.536 | 25.3 | 32.27 | 32.2 |
| 16.582 | 13.4 | 24.777 | 33.8 | 33.127 | 58.1 |
| 16.978 | 27.4 | 25.011 | 35.2 | 33.678 | 13 |
| 17.163 | 9.4 | 25.263 | 67 | 34.302 | 25.9 |
| 17.477 | 22.7 | 26.549 | 20.2 | 34.693 | 27.8 |
| 18.307 | 13.6 | 27.19 | 24.5 | 34.976 | 30.3 |
| 19.232 | 23.6 | 28.416 | 22.3 | | |

### (b) DSFF trihydrate

The title compound was prepared *in situ*, from circa 5 mg of the DSFF hexahydrate of Example 5(a), by heating at 60°C on the PXRD stage. PXRD:

| 2-Theta ° | Intensity % | 2-Theta ° | Intensity % | 2-Theta ° | Intensity % |
|---|---|---|---|---|---|
| 5.668 | 49.6 | 17.226 | 93.8 | 26.742 | 31.4 |
| 11.505 | 23 | 19.563 | 14.9 | 27.815 | 9.8 |
| 12.183 | 100 | 20.298 | 11.4 | 28.988 | 23.9 |
| 12.654 | 38.4 | 20.806 | 11.1 | 29.21 | 26 |
| 13.181 | 21.4 | 21.561 | 41.4 | 30.194 | 15.6 |
| 13.717 | 15.4 | 22.328 | 70.8 | 30.701 | 11.7 |
| 14.499 | 9.5 | 22.986 | 69.5 | 31.771 | 23.8 |
| 15.255 | 7.4 | 24.518 | 41.9 | 32.636 | 21.2 |
| 15.74 | 32.5 | 25.443 | 34.2 | 34.887 | 28.1 |
| 16.27 | 21.8 | | | | |

### Example 6 - DSFF hexahydrate

Fosfluconazole (26 mmols, 10g) was dissolved in a solution of sodium hydroxide (54 mmols, 2.17g final volume circa 20 ml) and heated to 70°C. lsopropyl alcohol (108ml), was added, in the temperature range 60 - 72°C, and the cloudy solution left to cool to provide the title compound as a slurry of crystals. A single crystal of DSFF hexahydrate was removed therefrom and mounted on a Bruker Diffractometer. The PXRD of Example 6 was consistent with that for Example 5(a).

## Claims

1. A process for the preparation of a stable hydrate mix of the disodium salt of fosfluconazole, or a composition comprising said compound, comprising:
a) providing a quantity of an aqueous mixture containing the disodium salt of fosfluconazole or a composition thereof in a suitable vessel in a freeze-drying apparatus;
b) reducing the temperature in the apparatus to bring about freezing and eutectic solidification;
c) reducing the pressure in the apparatus to below the saturation vapour pressure (SVP) of water over ice at the temperature of the ice;
d) maintaining the apparatus at a pressure below the SVP and, optionally, increasing the temperature in the apparatus to facilitate sublimation, until all of the ice has been sublimed;
e) maintaining the apparatus at the pressure and temperature conditions according to step d) until the desired water content has been obtained; and
f) either:
increasing the pressure in the apparatus to from 60% to 100% of atmospheric pressure (60.8 kPa to 101.3 kPa) and subsequently adjusting the temperature in the apparatus to from 5°C to 30°C;
or
adjusting the temperature in the apparatus to from 5°C to 30°C and subsequently increasing the pressure in the apparatus to from 60% to 100% of atmospheric pressure (60.8 kPa to 101.3 kPa).

2. A process according to claim 1, wherein the temperature of step b) is from -10°C to -50°C.

3. A process according to claim 1 or 2, wherein the temperature of step b) is from -20°C to -40°C.

4. A process according to any preceding claim, wherein the temperature of step b) is -30°C.

5. A process according to any preceding claim, wherein the pressure in step c) is from 1 Pa to 250Pa.

6. A process according to any preceding claim, wherein the pressure in step c) is from 2Pa to 125Pa.

7. A process according to any preceding claim, wherein the pressure in step c) is from 4Pa to 75Pa.

8. A process according to any preceding claim, wherein the temperature in step d) is from -50°C to 50°C.

9. A process according to any preceding claim, wherein the temperature in step d) is from -25°C to 0°C.

10. A process according to any preceding claim, wherein the temperature in step d) is -15°C.

11. A process according to any preceding claim, wherein the drying process is monitored by applying a standard pressure rise test in the freeze drying apparatus.

12. A process according to claim 11, wherein the drying process is monitored at intervals of one hour.

13. A process according to any preceding claim, wherein the duration of step (e) is from 0 to 100 hours.

14. A process according to any preceding claim, wherein the duration of step (e) is from 0 to 50 hours.

15. A process according to any preceding claim, wherein the duration of step (e) is from 6 to 30 hours.

16. A process according to claim 12, wherein the final water content of the stable hydrate mix is from 11% to 20% w/w.

17. A process according to claim 12 or claim 13, wherein the final water content of the stable hydrate mix is from 14% to 17% w/w.

18. A process according to claim 12 or claim 13, wherein the final water content of the stable hydrate mix is 15% w/w.

19. The disodium salt of fosfluconazole in the form of its trihydrate, its hexahydrate, or as a mixture of tri- and hexahydrates.

## Patentansprüche

1. Verfahren für die Herstellung einer stabilen Hydratmischung des Dinatriumsalzes von Fosfluconazol oder einer Zusammensetzung, die die genannte Verbindung umfasst, umfassend:
a) Bereitstellen einer Menge eines wässrigen Gemisches, das das Dinatriumsalz von Fosfluconazol oder eine Zusammensetzung davon enthält, in einem geeigneten Gefäß in einer Gefriertrocknungsapparatur;
b) Reduzieren der Temperatur in der Apparatur, um ein Gefrieren und ein eutektisches Festwerden zu bewirken;
c) Reduzieren des Drucks in der Apparatur unter den Sättigungsdampfdruck (SVP) von Wasser über Eis bei der Temperatur des Eises;
d) Halten der Apparatur bei einem Druck unter dem SVP und gegebenenfalls Erhöhen der Temperatur in der Apparatur zur Erleichterung einer Sublimation, bis das gesamte Eis sublimiert ist;
e) Halten der Apparatur bei den Druck- und Temperaturbedingungen entsprechend Schritt d), bis der gewünschte Wassergehalt erhalten wird; und
f) entweder:
Erhöhen des Drucks in der Apparatur von 60% bis 100% Atmosphärendruck (60,8 kPa bis 101,3 kPa) und anschließend Einstellen der Temperatur in der Apparatur auf 5°C bis 30°C;
oder
Einstellen der Temperatur in der Apparatur auf 5°C bis 30°C und anschließend Erhöhen des Drucks in der Apparatur auf 60% bis 100% Atmosphärendruck (60,8 kPa bis 101,3 kPa).

2. Verfahren nach Anspruch 1, wobei die Temperatur von Schritt b) -10°C bis -50°C ist.

3. Verfahren nach Anspruch 1 oder 2, wobei die Temperatur von Schritt b) -20°C bis -40°C ist.

4. Verfahren nach einem der vorangehenden Ansprüche, wobei die Temperatur von Schritt b) -30°C ist.

5. Verfahren nach einem der vorangehenden Ansprüche, wobei der Druck in Schritt c) 1 Pa bis 250 Pa ist.

6. Verfahren nach einem der vorangehenden Ansprüche, wobei der Druck in Schritt c) 2 Pa bis 125 Pa ist.

7. Verfahren nach einem der vorangehenden Ansprüche, wobei der Druck in Schritt c) 4 Pa bis 75 Pa ist.

8. Verfahren nach einem der vorangehenden Ansprüche, wobei die Temperatur in Schritt d) -50°C bis 50°C ist.

9. Verfahren nach einem der vorangehenden Ansprüche, wobei die Temperatur in Schritt d) -25°C bis 0°C ist.

10. Verfahren nach einem der vorangehenden Ansprüche, wobei die Temperatur in Schritt d) -15°C ist.

11. Verfahren nach einem der vorangehenden Ansprüche, wobei das Trocknungsverfahren überwacht wird, indem ein Standarddruckerhöhungstest in der Gefriertrocknungsapparatur angewendet wird.

12. Verfahren nach Anspruch 11, wobei das Trocknungsverfahren in Intervallen von einer Stunde überwacht wird.

13. Verfahren nach einem der vorangehenden Ansprüche, wobei die Dauer von Schritt (e) 0 bis 100 Stunden ist.

14. Verfahren nach einem der vorangehenden Ansprüche, wobei die Dauer von Schritt (e) 0 bis 50 Stunden ist.

15. Verfahren nach einem der vorangehenden Ansprüche, wobei die Dauer von Schritt (e) 6 bis 30 Stunden ist.

16. Verfahren nach Anspruch 12, wobei der Endwassergehalt der stabilen Hydratmischung 11 Gew.-% bis 20 Gew.-% ist.

17. Verfahren nach Anspruch 12 oder 13, wobei der Endwassergehalt der stabilen Hydratmischung 14 Gew.-% bis 17 Gew.-% ist.

18. Verfahren nach Anspruch 12 oder 13, wobei der Endwassergehalt der stabilen Hydratmischung 15 Gew.-% ist.

19. Dinatriumsalz von Fosfluconazol in der Form seines Trihydrats, seines Hexahydrats oder als Gemisch aus Tri- und Hexahydraten.

## Revendications

1. Procédé de préparation d'un mélange d'hydrates stables du sel disodique du fosfluconazole, ou d'une composition comprenant ledit composé, comprenant :
a) la fourniture d'une quantité d'un mélange aqueux contenant le sel disodique du fosfluconazole, ou d'une composition de ce composé, dans un récipient convenable, dans un appareil de lyophilisation ;
b) la réduction de la température dans l'appareil pour l'amener aux environs de la congélation et de la solidification eutectique ;
c) la réduction de la pression dans l'appareil au-dessous de la pression de vapeur à saturation (PVS) de l'eau, par rapport à la glace, à la température de la glace ;
d) le maintien de l'appareil à une pression inférieure à la PVS et, facultativement, l'augmentation de la température dans l'appareil pour faciliter la sublimation, jusqu'à ce que toute la glace se soit sublimée ;
e) le maintien de l'appareil dans les conditions de température et de pression selon l'étape d) jusqu'à ce que la teneur voulue en eau ait été obtenue ; et
f) soit :
l'augmentation de la pression dans l'appareil jusqu'à 60 % à 100 % de la pression atmosphérique (60,8 kPa à 101,3 kPa), puis réglage de la température de l'appareil entre 5°C et 30°C ;
soit
le réglage de la température dans l'appareil entre 5°C et 30°C, puis l'augmentation de la pression dans l'appareil jusqu'à 60 % à 100 % de la pression atmosphérique (60,8 kPa à 101,3 kPa).

2. Procédé selon la revendication 1, dans lequel la température à l'étape b) est comprise entre -10°C et -50°C.

3. Procédé selon la revendication 1 ou 2, dans lequel la température à l'étape b) est comprise entre -20°C et -40°C.

4. Procédé selon l'une quelconque des revendications précédentes, dans lequel la température à l'étape b) est de -30°C.

5. Procédé selon l'une quelconque des revendications précédentes, dans lequel la pression à l'étape c) est comprise entre 1 Pa et 250 Pa.

6. Procédé selon l'une quelconque des revendications précédentes, dans lequel la pression à l'étape c) est comprise entre 2 Pa et 125 Pa.

7. Procédé selon l'une quelconque des revendications précédentes, dans lequel la pression à l'étape c) est comprise entre 4 Pa et 75 Pa.

8. Procédé selon l'une quelconque des revendications précédentes, dans lequel la température à l'étape d) est comprise entre -50°C et 50°C.

9. Procédé selon l'une quelconque des revendications précédentes, dans lequel la température à l'étape d) est comprise entre -25°C et 0°C.

10. Procédé selon l'une quelconque des revendications précédentes, dans lequel la température à l'étape d) est de -15°C.

11. Procédé selon l'une quelconque des revendications précédentes, dans lequel l'étape de séchage est contrôlée par application d'un test standard d'élévation de pression dans l'appareil de lyophilisation.

12. Procédé selon la revendication 11, dans lequel l'étape de séchage est contrôlée à des intervalles d'une heure.

13. Procédé selon l'une quelconque des revendications précédentes, dans lequel la durée de l'étape (e) va de 0 à 100 heures.

14. Procédé selon l'une quelconque des revendications précédentes, dans lequel la durée de l'étape (e) va de 0 à 50 heures.

15. Procédé selon l'une quelconque des revendications précédentes, dans lequel la durée de l'étape (e) va de 6 à 30 heures.

16. Procédé selon la revendication 12, dans lequel la teneur finale en eau du mélange d'hydrates stables va de 11 % à 20 % poids/poids.

17. Procédé selon la revendication 12 ou la revendication 13, dans lequel la teneur finale en eau du mélange d'hydrates stables va de 14 % à 17 % poids/poids.

18. Procédé selon la revendication 12 ou la revendication 13, dans lequel la teneur finale en eau du mélange d'hydrates stables est de 15 % poids/poids.

19. Sel disodique du fosfluconazole sous la forme de son trihydrate, de son hexahydrate, ou d'un mélange de ses tri- et hexahydrates.
